# EUROPEAN PATENT SPECIFICATION

(11) **EP 3 371 569 B1**
(45) Date of publication and mention of the grant of the patent: **20.01.2021**
(21) Application number: 16862702.4
(22) Date of filing: 24.10.2016
(51) Int. Cl.: G01N 1/22, G01N 33/00, G01N 1/24

(54) **GAS SENSOR WITH A SEALABLE SAMPLING CHAMBER**
GASSENSOR MIT EINER ABDICHTBAREN PROBENKAMMER
CAPTEUR DE GAZ AVEC CHAMBRE D'ÉCHANTILLONNAGE POUVANT ÊTRE SCELLÉE

(30) Priority: 06.11.2015 US 201562251760 P
(43) Date of publication of application: 12.09.2018
(73) Proprietor: 3M Innovative Properties Company, St. Paul, MN 55133-3427 (US)
(72) Inventor: HU, Jia, Saint Paul, Minnesota 55133-3427 (US); TEMPLE, Daniel A., Cedar Park, Texas 78613 (US)
(74) Representative: Mathys & Squire
(86) International application number: PCT/US2016/058368
(87) International publication number: WO 2017/078950

(56) References cited:
- WO-A1-95/33202
- WO-A1-2008/070922
- WO-A1-2008/070922
- WO-A1-2015/019083
- CN-U- 203 025 149
- FR-A1- 2 924 811
- US-B1- 6 543 189
- US-B2- 8 741 120

## Description

### BACKGROUND

FR 2 924 811 relates to a system for monitoring an underground formation containing a least one gas, which comprises at least one device provided with a gas tapping means buried in the ground, a means for transferring the gas between said tapping means and a means for controlling a gas leak positioned at the surface and adapted for measuring the flow of the tapped gas and/or the composition of the tapped gas.

CN 203 025 149 U relates to a coal mine carbon monoxide sensor, wherein a handle is arranged at the upper part of a casing; a ventilation hole formed at the upper part of the casing is communicated with a filter membrane on the inner upper part of the casing; a gas-permeable membrane is arranged at the lower part of the filter membrane; a working electrode is arranged at the upper end of a cavity at the lower part of the gas-permeable membrane; the working electrode is connected with a first pin which is arranged at the lower end of the casing; a counter electrode and a reference electrode are arranged at the lower part of the cavity at intervals; an electrolyte solution is contained in the cavity; the counter electrode is connected with a second pin which is arranged at the lower end of the casing; and the reference electrode is connected with a third pin arranged at the lower end of the casing.

WO 2008/070922 discloses a flux chamber suitable for burial in the ground for long periods of time where it measures the subsurface gas fluxes; wherein the flux chamber comprises: an accumulation chamber and pipes extending above it into the atmosphere by which it can be flushed with air from the atmosphere from time to time; a gas sensor within the accumulation chamber to measure the concentration of the gas or gases of interest, and a filter or semi permeable membrane which is located at the lower end of the accumulation chamber to allow gases to enter the accumulation chamber but reject liquid water, dirt, and other undesirable substances.

Most gas sensors require gas permeable membranes, such as Polytetrafluoroethylene (PTFE), to prevent extreme moisture, water, or other elements from being in direct contact with the sensors as that may cause a false reading or damage. However, the membranes themselves are exposed to contamination and prone to degraded performance or damage if the water contains dust, dirt, salt, debris, or other contaminants which are often present in flooded underground spaces such as manholes. Accordingly, a need exists for a passive gas sensor assembly that can protect the gas sensor from water, moisture, and contaminants.

### SUMMARY

A first gas sensor assembly with a sealable chamber, consistent with the present invention, includes an enclosed chamber having an air passage, a gas sensor located within the chamber, and an air permeable membrane located within the chamber between the gas sensor and the air passage. The assembly also includes a tube having a first open end coupled to the chamber at the air passage and a second open end opposite the first end. The second end of the tube allows for air flow into the tube, and when a level of water in the tube rises to a particular level, air with positive pressure in the chamber prevents the water from penetrating the membrane.

A second gas sensor assembly with a sealable chamber, consistent with the present invention, includes a chamber having an air passage, a gas sensor located within the chamber, and an air permeable membrane located within the chamber between the gas sensor and the air passage. The assembly also includes a tube having a first open end coupled to the chamber at the air passage and a second open end opposite the first end. A float is located within the tube between a seal and a float seat and an air path. In use the float is raised by a water level inside tube and seals off membrane and gas sensor as the water rises above a certain level outside the assembly.

### BRIEF DESCRIPTION OF THE DRAWINGS

The accompanying drawings are incorporated in and constitute a part of this specification and, together with the description, explain the advantages and principles of the invention. In the drawings,
FIG. 1 is a block diagram illustrating a gas sensor assembly used to monitor an underground space;
FIG. 2 is a side view of a first embodiment of a gas sensor assembly with a sealable chamber;
FIG. 3 is a side view of the first embodiment with the chamber sealed;
FIG. 4 is a side view of a second embodiment of a gas sensor assembly with a sealable chamber;
FIG. 5 is a side view of the second embodiment with the chamber sealed;
FIG. 6 is a side view of a third embodiment of a gas sensor assembly with a sealable chamber; and
FIG. 7 is a side view of the third embodiment with the chamber sealed.

### DETAILED DESCRIPTION

Embodiments of the invention include assemblies to package sensors that monitor gases, for example methane, carbon monoxide, sulfide, and others, in underground infrastructures by placing those sensors that are prone to water damage in a sealable sampling chamber. In those systems, the chamber can be open to allow the sensors to sample and monitor the air for gases and can be passively closed to protect the sensors from water and contaminants.

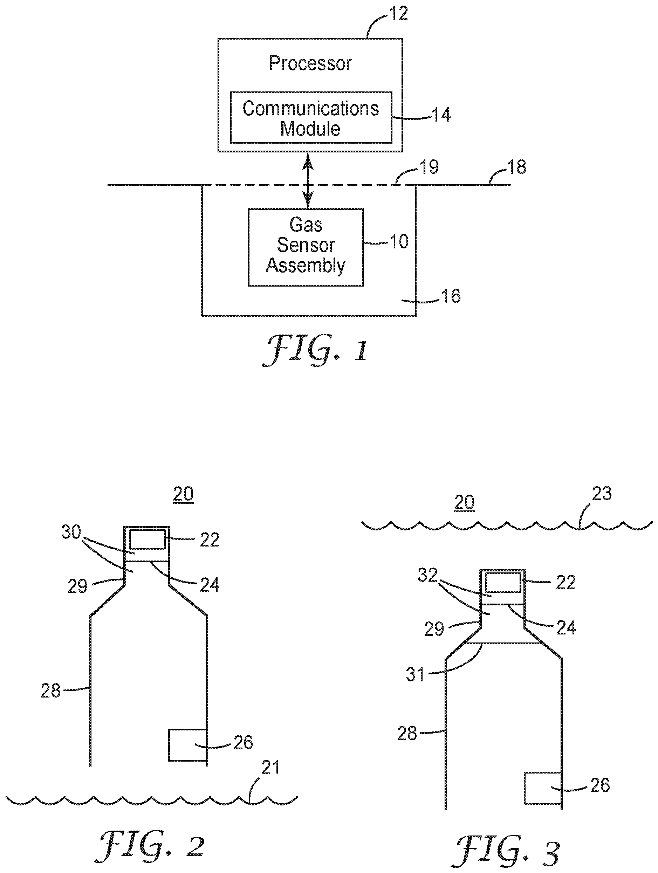

FIG. 1 is a block diagram illustrating a gas sensor assembly used to monitor an underground space. A gas sensor assembly 10 is located within an underground space 16 below ground or grade level 18. Gas sensor assembly 16 can be electrically coupled to a processor 12, which can provide power to gas sensor assembly 16 and receive sensor signals from it. A communications module 14 within or associated with processor 12 can be used to send signals related to the sensor signals received from gas sensor assembly 16. For example, communications module 14 can send the signals via communications protocols, including wireless communications protocols, for transmitting the signals over the Internet or other networks. An opening 19, such as a manhole, can be used to physically access gas sensor assembly 16.

FIG. 2 is a side view of a gas sensor assembly 20 with a sealable chamber, and FIG. 3 is a side view of gas sensor assembly 20 with the chamber sealed. Assembly 20 includes a chamber 29 having an air passage to a downward tube 28 and having a closed end opposite tube 28. Tube 28 has a first open end at chamber 29 and a second open end opposite the first open end. A gas sensor 22 is located within chamber 29, and an air permeable membrane 24 is located between gas sensor 22 and the air passage to tube 28. In use, air pressure inside tube 28 builds as the water level rises, for example from level 21 to level 23. Positive air pressure 32 inside chamber 29 and tube 28 keeps the water level inside the tube lower than outside water level 23 and maintains an air pocket at the top of tube 28 surrounding membrane 24 and gas sensor 22 in chamber 29, thus preventing the water from penetrating membrane 24 and reaching gas sensor 22. This pressure differential, inside and outside, protects membrane 24 and gas sensor 22. Assembly 20 can optionally include a water level sensor 26 in tube 28 to monitor and provide a signal related to a water level in tube 28.

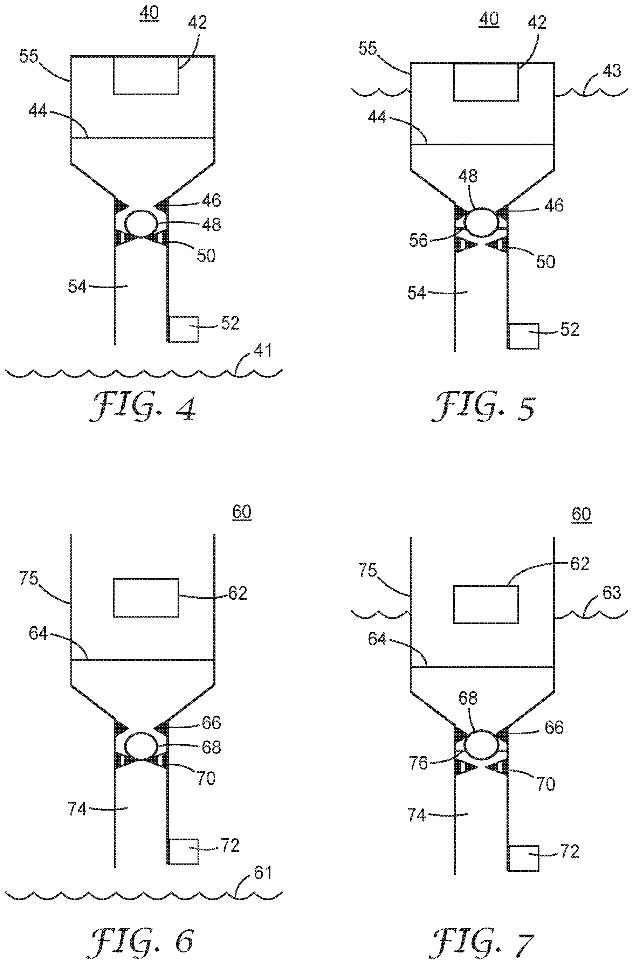

FIG. 4 is a side view of a gas sensor assembly 40 with a sealable chamber, and FIG. 5 is a side view of gas sensor assembly 40 with the chamber sealed. Assembly 40 includes a chamber 55 having an air passage to a downward tube 54 and having a closed end opposite tube 54. Tube 54 has a first open end at chamber 55 and a second open end opposite the first open end. A gas sensor 42 is located within chamber 55, and an air permeable membrane 44 is located between gas sensor 42 and the air passage to tube 54. A float 48 is located within tube 54 between a seal 46 and a float seat and air path 50. In use, float 48 is raised by water level 56 inside tube 54 and seals off membrane 44 and gas sensor 42 as the water rises above a certain level outside assembly 40, for example from level 41 to level 43. Assembly 40 can optionally include a water level sensor 52 adjacent tube 54 to monitor and provide a signal related to a water level outside and in contact with tube 54.

FIG. 6 is a side view of a gas sensor assembly 60 with a sealable chamber, and FIG. 7 is a side view of gas sensor assembly 60 with the chamber sealed. Assembly 60 includes a chamber 75 having an air passage to a downward tube 74 and having an open end opposite tube 74. Tube 74 has a first open end at chamber 75 and a second open end opposite the first open end. A gas sensor 62 is located within chamber 75, and an air permeable membrane 64 is located between gas sensor 62 and the air passage to tube 74. A float 68 is located within tube 74 between a seal 66 and a float seat and air path 70. In use, float 68 is raised by water level 76 inside tube 74 and seals off membrane 64 and gas sensor 62 as the water rises above a certain level outside assembly 60, for example from level 61 to level 63. Assembly 60 can optionally include a water level sensor 72 adjacent tube 74 to monitor and provide a signal related to a water level outside and in contact with tube 74. The open end at the top of chamber 75 allows gas sensor 62 to continue to monitor the environment even when there is a certain level of water above gas sensor 62, for example level 63.

The following are exemplary materials, components, and configurations for the gas sensor assemblies described herein.

The gas sensors can be implemented with the following: the Synkera Technologies, Inc. UltraKera 729 and the Figaro USA Inc. TGS2611 products for detecting methane; the Synkera Technologies, Inc. MikroKera 727 product for detecting hydrogen sulfide; and the Figaro USA Inc. TGS3870 and the SGX Sensortech MiCs-5524 products for detecting carbon monoxide.

The chamber and tube can be composed of solid metal or plastic sides, between the top of the chamber and the second end of the tube, to prevent water from entering the assembly through the sides. The chamber and tube can have a round, square, rectangular, or other cross-sectional shape when viewed from the open end of the tube or the top of the chamber. The assemblies can be physically mounted within an underground space, such as a manhole or vault, to provide the monitoring.

The gas sensors and water level sensors are electrically coupled to the processor to provide sensor signals such as a signal relating to gas detected by the gas sensor and a signal relating to a water level detected by the water level sensor. The processor can be configured to process those received sensor signals. Based upon a signal from the gas sensor the processor via the communications module can send an alert or warning signal. Based upon a signal from the water level sensor, the processor can be configured to turn off power to the gas sensor. The processor and communications module are shown remote from the gas sensor assemblies but can optionally be located within the assemblies.

## Claims

1. A gas sensor assembly (10, 20) with a sealable chamber, comprising:
a chamber (29) having an air passage, wherein the chamber is enclosed around the air passage;
a gas sensor (22) located within the chamber;
an air permeable membrane (24) located within the chamber between the gas sensor and the air passage;the gas sensor assembly being **characterized by**
a tube (28) having a first open end coupled to the chamber at the air passage and having a second open end opposite the first end,
wherein the second end of the tube allows for air flow into the tube, and when a level of water in the tube rises to a particular level, air with positive pressure in the chamber prevents the water from penetrating the membrane.

2. The gas sensor assembly (10, 20) of claim 1, further comprising a water level sensor (26) located adjacent the tube (28).

3. A gas sensor assembly (10, 40, 60) with a sealable chamber, comprising:
a chamber (55, 75) having an air passage;
a gas sensor (42, 62) located within the chamber;
an air permeable membrane (44, 64) located within the chamber between the gas sensor and the air passage; the gas sensor assembly being **characterized by**
a tube (54, 74) having a first open end coupled to the chamber at the air passage and having a second open end opposite the first end; and
a float (48, 68) located within the tube between a seal (46, 66) and a float seat and an air path (50, 70), wherein, in use, the float (48) is raised by a water level (56) inside the tube (54) and seals off the membrane (44, 64) and the gas sensor (42, 62) as the water rises above a certain level outside the assembly (40, 60).

4. The gas sensor assembly (40) of claim 3, wherein the chamber (55) is enclosed around the air passage.

5. The gas sensor assembly (60) of claim 3, wherein the chamber (75) has an open end opposite the air passage.

6. The gas sensor assembly (40, 60) of claim 3, further comprising a water level sensor (52, 72) located adjacent the tube (54, 74).

## Patentansprüche

1. Eine Gassensorbaugruppe (10, 20) mit einer abdichtbaren Kammer, umfassend:
eine Kammer (29) mit einem Luftdurchlass, wobei die Kammer um den Luftdurchlass abgegrenzt ist;
einen Gassensor (22), der sich innerhalb der Kammer befindet;
eine luftdurchlässige Membran (24), die sich innerhalb der Kammer zwischen dem Gassensor und dem Luftdurchlass befindet; wobei die Gassensorbaugruppe **gekennzeichnet ist durch** ein Rohr (28) mit einem ersten offenen Ende, das mit der Kammer an dem Luftdurchlass verbunden ist, und mit einem zweiten offenen Ende gegenüber dem ersten Ende,
wobei das zweite Ende des Rohrs einen Luftstrom in das Rohr ermöglicht, und wenn ein Wasserpegel in dem Rohr auf einen bestimmten Pegel ansteigt, Luft mit positivem Druck in der Kammer verhindert, dass das Wasser in die Membran eindringt.

2. Die Gassensorbaugruppe (10, 20) nach Anspruch 1, ferner umfassend einen Wasserstandssensor (26), der sich neben dem Rohr (28) befindet.

3. Eine Gassensorbaugruppe (10, 40, 60) mit einer abdichtbaren Kammer, umfassend:
eine Kammer (55, 75) mit einem Luftdurchlass;
einen Gassensor (42, 62), der sich innerhalb der Kammer befindet;
eine luftdurchlässige Membran (44, 64), die sich innerhalb der Kammer zwischen dem Gassensor und dem Luftdurchlass befindet; wobei die Gassensorbaugruppe **gekennzeichnet ist durch**
ein Rohr (54, 74) mit einem ersten offenen Ende, das mit der Kammer an dem Luftdurchlass verbunden ist, und mit einem zweiten offenen Ende gegenüber dem ersten Ende; und
einen Schwimmer (48, 68), der sich innerhalb des Rohrs zwischen einer Dichtung (46, 66) und einem Schwimmersitz und einem Luftpfad (50, 70) befindet, wobei der Schwimmer (48) im Gebrauch **durch** einen Wasserpegel (56) innerhalb des Rohrs (54) angehoben wird und die Membran (44, 64) und den Gassensor (42, 62) abdichtet, wenn das Wasser über einen bestimmten Pegel außerhalb der Baugruppe (40, 60) steigt.

4. Die Gassensorbaugruppe (40) nach Anspruch 3, wobei die Kammer (55) um den Luftdurchlass abgegrenzt ist.

5. Die Gassensorbaugruppe (60) nach Anspruch 3, wobei die Kammer (75) ein offenes Ende gegenüber dem Luftdurchlass aufweist.

6. Die Gassensorbaugruppe (40, 60) nach Anspruch 3, ferner umfassend einen Wasserstandssensor (52, 72), der sich neben dem Rohr (54, 74) befindet.

## Revendications

1. Ensemble de capteur de gaz (10, 20) avec une chambre scellable, comprenant :
une chambre (29) présentant un passage d'air, dans lequel la chambre est enfermée autour du passage d'air ;
un capteur de gaz (22) situé à l'intérieur de la chambre ;
une membrane perméable à l'air (24) située à l'intérieur de la chambre entre le capteur de gaz et le passage d'air ; l'ensemble de capteur de gaz étant **caractérisé par** un tube (28) doté d'une première extrémité ouverte couplée à la chambre au niveau du passage d'air et doté d'une deuxième extrémité ouverte opposée à la première extrémité,
dans lequel la deuxième extrémité du tube permet un écoulement d'air dans le tube, et lorsqu'un niveau d'eau dans le tube s'élève à un niveau particulier, l'air à pression positive dans la chambre empêche l'eau de pénétrer dans la membrane.

2. Ensemble de capteur de gaz (10, 20) selon la revendication 1, comprenant en outre un capteur de niveau d'eau (26) situé de manière adjacente au tube (28).

3. Ensemble de capteur de gaz (10, 40, 60) avec une chambre scellable, comprenant :
une chambre (55, 75) dotée d'un passage d'air ;
un capteur de gaz (42, 62) situé à l'intérieur de la chambre ;
une membrane perméable à l'air (44, 64) située à l'intérieur de la chambre entre le capteur de gaz et le passage d'air ; l'ensemble de capteur de gaz étant **caractérisé par** un tube (54, 74) doté d'une première extrémité ouverte couplée à la chambre au niveau du passage d'air et doté d'une deuxième extrémité ouverte opposée à la première extrémité ; et
un flotteur (48, 68) situé à l'intérieur du tube entre un joint d'étanchéité (46, 66) et un siège de flotteur et un passage d'air (50, 70), dans lequel, en cours d'utilisation, le flotteur (48) est levé par un niveau d'eau (56) à l'intérieur du tube (54) et obstrue la membrane (44, 64) et le capteur de gaz (42, 62) lorsque l'eau s'élève au-dessus d'un certain niveau à l'extérieur de l'ensemble (40, 60).

4. Ensemble capteur de gaz (40) selon la revendication 3, dans lequel la chambre (55) est enfermée autour du passage d'air.

5. Ensemble capteur de gaz (60) selon la revendication 3, dans lequel la chambre (75) présente une extrémité ouverte opposée au passage d'air.

6. Ensemble de capteur de gaz (40, 60) selon la revendication 3, comprenant en outre un capteur de niveau d'eau (52, 72) situé de manière adjacente au tube (54, 74).
